# EUROPÄISCHE PATENTSCHRIFT

(11) **EP 1 682 227 B1**
(45) Veröffentlichungstag und Bekanntmachung des Hinweises auf die Patenterteilung: **04.06.2008**
(21) Anmeldenummer: 04818136.6
(22) Anmeldetag: 09.11.2004
(51) Int. Cl.: A61Q 1/10, A61Q 5/00

(54) **VERFAHREN ZUR HERSTELLUNG EINER MASCARA MIT FASERBESTANDTEILEN**
PROCESS FOR MANUFACTURING A MASCARA WITH FIBER COMPONENTS
PROCEDE POUR LA FABRICATION DU MASCARA A CONSTITUANTS A BASE DE FIBRES

(30) Priorität: 10.11.2003 DE 10353486
(43) Veröffentlichungstag der Anmeldung: 26.07.2006
(73) Patentinhaber: Coty B.V., 2031 CC Haarlem (NL)
(72) Erfinder: LOGINOVA, Yelena, Bronx NY, New York 10467 (US); MACCHIO, Ralph, Sparta, New Jersey 07971 (US); FARER, Alan, Kinnelon, New Jersey 07405 (US); KOLMAN, Toni, Hillsborough, New Jersey 08844 (US)
(74) Vertreter: Ziebig, Marlene
(86) Internationale Anmeldenummer: PCT/EP2004/012855
(87) Internationale Veröffentlichungsnummer: WO 2005/044204

(56) Entgegenhaltungen:
- EP-A- 1 172 078
- WO-A-02/49601
- US-A1- 2002 098 217
- US-A1- 2002 142 014

## Beschreibung

Die Erfindung betrifft ein Verfahren zur Herstellung einer Mascara mit Faserbestandteilen.

Die Einbeziehung von Fasern in Mascara-Zusammensetzungen ist bereits bekannt. Der Hauptvorteil von Mascara mit Fasern darin ist die Verlängerung der Wimpern sowie ein gewisser Volumeneffekt. Der Nachteil einer solchen Mascara ist dessen schlechte Gebrauchseigenschaft während der normal täglichen Einsatzzeit (üblicherweise 8 bis 12 Std./Tag) . Es gibt drei gebräuchliche Merkmale für die Gebrauchseigenschaften eines Faser-Mascara, die sich in der Praxis gezeigt haben:
1) Ablösung der Fasern von den Wimpern, was unzureichende Haftungseigenschaften der gewählten Filmbildner anzeigt;
2) Abschuppung (flaking), was ein Ungleichgewicht zwischen den Haftungseigenschaften und der Brüchigkeit des Filmes auf den Wimpern anzeigt;
3) Trocknungszeit, die zu kurz oder zu lang ist. Ist sie zu lang, können schnelle nachfolgende Auftragungsvorgänge Fasern von den Wimpern wieder ablösen. Wenn die Trocknungszeit zu kurz ist, kann keine klumpenfreie Auftragung erfolgen und jeder Versuch, die Wimpern zu vereinzeln, kann zu einer Ablösung der Faser von den Wimpern führen.

Jedes Merkmal für sich oder zusammen minimiert die Schönheitseigenschaften dieser Mascara und dessen Sicherheit im Falle, dass Bruchstücke in die Augen fallen und diese gerieben werden, um die Fremdkörper zu entfernen. Alle Nachteile abgelöster Fasern machen dieses Produkt für den Verbraucher unerwünscht.

Entsprechend der ausgewiesenen Formel verwenden alle bisher bekannten Formulierungen von Faser-Mascara die wasserlöslichen oder wasserdispergierbaren Polymere/Filmbilder allein oder in Kombination mit den öllöslichen oder öldispergierbaren Filmbildnern/Polymeren (US 6,491,931; US-Anmeldung 2002/0,110,571; US-Anmeldung 2002/0,192,251; US 6, 482, 400) .

Die meisten wasserlöslichen.Filmbildner sind am effektivsten im pH-Bereich von 4 bis 6. Im Allgemeinen liegt der pH-Bereich für die Mascara zwischen 7 und 8, wodurch die Eigenschaften des filmbildenden Polymeren modifiziert werden. Auch die meisten dieser Polymeren/Filmbildner sind hygroskopisch und erfordern das Vorhandensein von speziellen Mitteln, um ihre hygroskopischen Eigenschaften zu verringern und die Abnutzung zu verbessern.

Im Verfahrensablauf gibt es eine Stufe bei der Gemischherstellung, die eine besondere Bedeutung für eine Mascara mit Faser hat - die Steuerung und Aufrechterhaltung der Faserdispersion, die die gleichbleibende Qualität der Produktleistungsmerkmale sichert. Das in der US 2002/0,110,571 beschriebene Verfahren bezieht sich darauf, dass die Faserdispersion bei 85 bis 90 °C in die Wasserphase nach den Verdickungsstoffen, Pigmenten und den anderen Bestandteilen der Wasserphase zugegeben wird.

EP 1 172 078 beschreibt eine Mascara, die Elastofasern enthält. Die Herstellung erfolgt durch Mischen der einzelnen Komponenten.

Die Aufgabe ist die Bereitstellung eines Verfahrens zur Herstellung der Mascara.

Die Mascara der vorliegenden Erfindung umfasst
0,1 bis 10 Gew-% wenigstens eines öllöslichen oder öldispergierbaren Polymeren oder Copolymeren,
0,3 bis 10 Gew-% einer natürlichen oder synthetischen Faser mit einer mittleren Länge von 3 bis 6 mm,
10 bis 30 Gew-% eines natürlichen oder synthetischen Wachses der bei 25 °C fest wird,
1 bis 10 Gew-% eines bei 18 °C und darüber flüssigen synthetischen Wachses,
0,5 bis 10 Gew-% anorganische Pigmente, organische Farbstoffe und Gemische davon,
40 bis 80 Gew-% Wasser
und den Rest bis zu 100 Gew-% kosmetische Hilfsstoffe, Wirkstoffe und Gemische davon,
wobei die Mascara keine hydrophilen Polymere, Filmbildner, Verdickungsmittel oder Tone enthält, die wasserlöslich oder wasserdispergierbar sind,
und wobei alle Prozentangaben auf das Gewicht der Gesamtzusammensetzung bezogen sind.

Die Erfindung führt zu einer Faser-Mascara-Zusammensetzung mit allein hydrophoben Filmbildner(n) und einer sehr guten Faserhaltekraft. Beim Verfahren zur Herstellung der Mascara werden die Fasern mit dem Polymeren als Zwischenstufe kalt vorgemischt, und nachfolgend wird diese Vormischung der Emulsion zugegeben.

Die Faser in der Mascara hat vorzugsweise eine durchschnittliche Länge von 4 bis 4,5 mm.

Die Fasern können ausgewählt werden unter Polyesterfasern, Rayonfasern, Nylonfasern, Baumwollfasern, Teflonfasernn und sind vorzugsweise Lycra^{®}-Fasern.

Das öllösliche oder öldispergierbare Polymere ist ausgewählt aus der Gruppe, bestehend aus Copolymeren von Maleinsäureanhydrid, Isopropylmaleate und Olefinmonomeren mit 30 bis 45 Kohlenstoffatomen; Copolymeren von Vinylpyrrolidon und langkettigen alpha-Olefinen; Copolymeren von Adipinsäure mit Fumarsäure, Phthalsäure und Tricyclodecane dimethiconol; Copolymeren von Adipinsäure, Cyclohexane-dimethanol, Maleinsäureanhydrid, Neopentylglycol und Trimellithsäure-anhydrid-Monomeren; Copolymeren von Adipinsäure und PPG-10-Monomeren; Polyethylen; Butadien/Isopren-Copolymeren; Copolymeren von Ethyl- oder Butylestern von PVM/MA-Copolymeren; Tricontanyl PVP; C20-40 Acid (and) Polyethylene; PVP/Eicosene; Bis-Diglyceryl Polyacyladipate-1; Polyvinyl-octadecylether; und Gemischen davon; oder ein anderes öl-lösliches oder öl-dispergierbares Polymeres, Filmbildner oder Ö1-verdickendes/Ö1-gelierendes Mittel.

Besonders bevorzugt sind Tricontanyl PVP, C20-40 Acid (and) Polyethylene sowie PVP/Eicosene (PVP=Polyvinylpyrrolidon). Das öllösliche oder öldispergierbare Polymere wird vorzugsweise im Bereich von 0,5 bis 7 Ges-% eingesetzt.

Hilfsmittel sind Duftstoffe, Markierungsmittel, Vitamine, Antioxidationsmittel und Schutzmittel, die gegen grammpositive und gramm-negative Bakterien wirksam sind, Hefen und Formgebungsmittel. Weitere Gelierungs-/Verdickungsmittel für die Ölsphase sind, zusätzlich zu den oben genannten öllöslichen oder -dispergierbaren Polymeren, solche Produkte wie C20-40 Acide (and)Polyethylene, Decene/Butene Copolymer, Disteardimonium Hectorite.

Oberflächenaktive Mittel sind z.B. Lecithin, Sorbitan Sesquioleate oder ein beliebiges anderes mit niedrigem HLB für die W/O-Emulsion. Für W/O-Emulsionen sind bevorzugte oberflächenaktive Mittel z.B. Polysorbate 20, Oleth-20 oder ein beliebiges anderes mit hohen HLB.

Anorganische Pigmente sind z.B. Eisenoxide, Titaniumdioxid, Ultramarine, Glimmer, Chromoxid, Chromhydroxid. Auch organische Farbstoffe können eingesetzt werden, wie FD&C Red 40, FD&C Yellow 5, FD&C Blue 1 und ihre Lacke sowie Green D&C 5, mit Carmine.

Die Mascara der vorliegenden Erfindung hat gute Eignenschaften in Bezug auf die Verbesserung des Auftragens, der Verlängerung, der volumenvergrößerung und des Biegens der Wimpern. Die Zusammensetzung kann allein auf die Wimpern aufgetragen, werden, oder sie kann mit einer anderen Mascara noch überschichtet werden, die auf einer regulären Emulsion beruht, und zwar der Kategorie "wasserfest" oder der Gel-Kategorie, d.h. als Unterschicht- oder Deckschicht-Mascara. Die Zusammensetzung kann auch als Augenbrauenfüllstoff verwendet werden. Die Zusammensetzung hat eine einzigartige klumpenfreie Faserhaltekraft.

Natürliche oder synthetische Wachse, die bei 25 °C fest werden, sind beispielsweise Bienenwachs, Ozokerit, Carnaubawachs, Candellilawachs, Wollwachs, Hartparaffin, Ceresin, Siliconwachs, Polyethylenglycol- oder -glycolesterwachse.

Ein bevorzugter Wachs, der bei 18°C und darüber flüssig wird, ist z.B. ein entsprechender Polyethylenwachs, ein synthetischer Wachs (INCI: Synthetic Wax) oder VP/Hexadecene Copolymer.

Das Verfahren zur Herstellung der Faser-Mascara, wie es in der vorliegenden Erfindung beschrieben ist, sieht eine steuerbare Trennung der Faserdispersionsherstellung in einer klaren Basisformulierung (flüssiger synthetischer Wachs) vor. Der erfindungsgemäße Prozess zur Herstellung der Mascara umfasst das Vermischen einer Ölphase, die Wachse, Öle, Pigmente und wenigstens ein öllösliches oder öldispergierbares Polymeres oder Copolymeres enthält, mit einer Wasserphase bei 65 bis 78 °C bis zur vollständigen Emulgierung, und weiterhin ein Vermischen des homogenen Gemisches aus Fasern und flüssigem synthetischen Wachs, flüssigem Polyethylen oder einem anderen flüssigen polymeren Filmbildner unter Rühren und bei 50 bis 70 °C mit der Emulsion, mit der das Fasergemisch hergestellt wurde. Diese Herstellung der Faseremulsion erfolgt durch Rühren bei 12 bis 20 U/min bei 18 bis 25 °C.

Dabei wird die Faseremulsion/dispersion ohne Zusatz von Alkohol hergestellt.

Vorteilhaft ist auch die Abwesenheit wasserlöslichen oder - dispergierbaren Polymeren, Filmbildnern, Verdickungsmitteln oder Tonen.

Durch das vorliegende Verfahren im Bereich von Raumtemperatur (18 bis 25 °C) und mit sehr geringen Rührkräften über einen Zeitraum von 5 bis 15 Min. erhält man eine sehr stabile Faserdispersion mit einer ausgezeichneten Faserverteilung.

Die Haftung der erfindungsgemäßen Mascara wurde mit Marktprodukten in Vergleichsversuchen getestet. Dabei wurde gefunden, dass die Haftung um 15-20 % verbessert wurde und ausgezeichnete Aussagen in Consumer-Tests zur Faserverteilung und Auftragung der Mascara vorlagen.

Die Emulsion insgesamt hat einen pH-Wert von 7-8 und ist damit sehr gut verträglich für den Anwender.

Nachfolgend wird die Erfindung detailliert durch Beispiele beschrieben. Alle Prozentangaben sind Gewichtsprozent, sofern nichts anderes angegeben ist.

### Beispiel 1 Mascara I

| **PHASE A** | |
|---|---|
| Carnaubawachs | 4 |
| Ozokerite | 2 |
| Paraffin | 4 |
| Stearinsäure | 4 |
| Sorbitan Sesquioleate | 1 |
| Bienenwachs | 6 |
| Tricontanyl PVP | 2 |

| **PHASE B** | |
|---|---|
| Pigment Black Oxide | 8 |

| **PHASE C** | |
|---|---|
| Simethicone | 0,2 |

| **PHASE D** | |
|---|---|
| Wasser | q.s. ad 100 |
| Triethanolamin | 1,5 |
| Propylenglycol | 1,8 |

| **PHASE E** | |
|---|---|
| Synthetischer Wachs (flüssig bei 20°C) | 5 |
| Rayonfaser | 1 |

| **PHASE F** | |
|---|---|
| Vitamin | 1,2 |

| **PHASE G** | |
|---|---|
| Konservierungsmittel | 1,0 |

Die Ölphase (A) wird auf 80 bis 85 °C erhitzt. Die Pigmente von Phase (B) werden hinzugegeben und für 20 Min. bei 2500 U/min homogenisiert. Danach wird Simeticone (C) hinzugegeben. Die Wasserphase (D) wird auf 75 °C erhitzt und mit 200 bis 300 U/min gemischt. Beide Phasen werden miteinander vereinigt und für 20 Min. bei 2500 U/min homogenisiert. Dabei wird die Temperatur bei 65 bis 70 °C gehalten. Anschließend wird auf 60 bis 55 °C abgekühlt.

Die Fasern werden bei 20 °C mit 15 bis U/min über 5 bis 10 Min. mit dem synthetische Wachs von Phase (E) vorgemischt. Das Gemisch wird in den Hauptbehälter bei 60 bis 65 °C gegeben. Danach werden die Phasen F und G bei niedrigerer Temperatur zugesetzt.

Der wichtige Schritt dieses Verfahrens ist die Behandlung der Fasern mit dem flüssigen synthetischen Wachs, um die Fasern zu ordnen und zu trennen und dabei eine klumpenfreie Faserdispersion zu erhalten. Dies ist ein "Kalt"-Verfahren, das als Zwischenstufe vor der Einarbeitung der Fasern in die Emulsion erfolgt. Diese Behandlung gestattet die Beibehaltung einer guten Faserdispersion. Die Zugabe der Faserphase zu dem anderen Gemisch erfolgt mit einem Rührer mit moderatem Rührverhalten bei 65 °C.

### Beispiel 2 Mascara II

| **PHASE A:** | |
|---|---|
| Bienenwachs | 5 |
| Montanwachs | 3 |
| Candelilla | 2 |
| Stearinsäure | 5 |
| Lecithin | 1 |
| Mikrokristalliner Wachs | 4 |
| C20-40 Acid (and) Polyethylene | 3 |

| **PHASE B:** | |
|---|---|
| Pigment Black Oxide | 9 |

| **PHASE C:** | |
|---|---|
| Wasser | q.s. ad 100 |
| Triethanolamin | 1,7 |
| Glycerin | 1 |

| **PHASE D:** | |
|---|---|
| VP/Hexadecene Copolymer | 6 |
| Rayonfaser | 2 |
| Polyurethan - "Lycra"-faser | 1 |

| **PHASE F:** | |
|---|---|
| Vitamin | 1 |

| **PHASE G:** | |
|---|---|
| Konservierungsmittel | 0,8 |

Die Ölphase wurde hergestellt und ebenso die Wasserphase. Beide wurden wie oben beschrieben zusammengeführt. Dann erfolgte die Vormischung der Faser mit VP/Hexadecene Copolymer, und dieses Vorgemisch wurde der Hauptemulsion zugeführt entsprechend den Ausführungen zum Beispiel 1.

## Patentansprüche

1. Verfahren zur Herstellung eines Mascara mit Faserbestandteilen, **dadurch gekennzeichnet, dass** eine Ölphase, die Wachse, Öle, Pigmente und wenigstens ein öllösliches oder öldispergierbares Polymer oder Copolymer enthält, mit einer Wasserphase bei 65-78 °C bis zur vollständigen Emulgierung vermischt wird und ein bei 18 bis 25°C unter Rühren bei 12-20 U/min hergestelltes homogenes, alkoholfreies Gemisch aus Fasern und flüssigem synthetischen Wachs, flüssigem Polyethylen oder Gemischen davon unter Rühren bei 50-70 °C mit der Emulsion vermischt wird.

2. Verfahren nach Anspruch 1, **dadurch gekennzeichnet, dass** das wenigstens eine öllösliche oder öldispergierbare Polymer oder Copolymer ausgewählt ist aus der Gruppe bestehend aus Copolymeren von Maleinsäureanhydrid, Isopropylmaleate und Olefinmonomeren mit 30 bis 45 Kohlenstoffatomen; Copolymeren von Vinylpyrrolidon und langkettigen alpha-Olefinen; Copolymeren von Adipinsäure mit Fumarsäure, Phthalsäure und Tricyclodecane dimethiconol; Copolymeren von Adipinsäure, Cyclohexane-dimethanol, Maleinsäureanhydrid, Neopentylglycol und Trimellithsäure-anhydrid-Monomeren; Copolymeren von Adipinsäure und PPG-10-Monomeren; Polyethylen; Butadien/Isopren-Copolymeren; Copolymeren von Ethyl- oder Butylestern von PVM/MA-Copolymeren; Tricontanyl PVP; C20-40 Acid (and) Polyethylene; PVP/Eicosene; Bis-Diglyceryl Polyacyladipate-1; Polyvinyl-octadecylether; und Gemischen davon.

3. Verfahren nach Anspruch 2, **dadurch gekennzeichnet, dass** das wenigstens eine öllösliche oder öldispergierbare Polymer oder Copolymer aus der Gruppe ausgewählt ist, bestehend aus Tricontanyl PVP, C20-40 Acid (and) Polyethylene, PVP/Eicosene und Gemischen davon.

4. Verfahren nach einem der Ansprüche 1 bis 3, **dadurch gekennzeichnet, dass** als Fasern Polyesterfasern, Rayonfasern, Nylonfasern, Baumwollfasern, Teflonfasern und vorzugsweise Lycra®-Fasern eingesetzt werden.

5. Verfahren nach einem der Ansprüche 1 bis 4, **dadurch gekennzeichnet, dass** als Fasern solche mit einer mittleren Länge von 4 bis 4,5 mm eingesetzt werden.

6. Verfahren nach einem der Ansprüche 1 bis 5, **dadurch gekennzeichnet, dass** die in der Ölphase eingesetzten Wachse solche natürlichen oder synthetischen Wachse sind, die bei 25°C und darunter fest sind, vorzugsweise Bienenwachs, Ozokerit, Carnaubawachs, Candellilawachs, Wollwachs, Hartparaffin, Ceresin, Siliconwachs, Polyethylenglycol- oder -glycolesterwachse.

## Claims

1. A method for producing a mascara containing fibre components, wherein an oil phase comprising waxes, oils, pigments and at least one oil-soluble or oil-dispersible polymer or copolymer is mixed with an aqueous phase at 65-78°C until complete emulsification, and the aforesaid emulsion is mixed at 50-70°C by stirring with a homogeneous, alcohol-free mixture of fibres and liquid synthetic wax, liquid polyethylene or mixtures thereof, which has been prepared at between 18 and 25°C and stirring at 12-20 rpm.

2. The method according to claim 1, wherein said at least one oil-soluble or oil-dispersible polymer or copolymer is selected from the group consisting of copolymers of maleic anhydride, isopropylmaleate and olefin monomers having between 30 and 45 carbon atoms; copolymers of vinylpyrrolidone and long-chain alpha-olefins; copolymers of adipic acid with fumaric acid, phthalic acid and tricyclodecane dimethiconol; copolymers of adipic acid, cyclohexanedimethanol, maleic anhydride, neopentyl glycol and trimellitic anhydride monomers; copolymers of adipic acid and PPG-10 monomers; polyethylene; butadiene/isoprene copolymers; copolymers of ethyl esters or butyl esters of PVM/MA copolymers; Tricontanyl PVP; C20-40 Acid (and) Polyethylene; PVP/Eicosene; Bis-Diglyceryl Polyacyladipate-1; Polyvinyl Octadecyl Ether; and mixtures thereof.

3. The method according to claim 2, wherein said at least one oil-soluble or oil-dispersible polymer or copolymer is selected from the group consisting of Tricontanyl PVP, C20-40 Acid (and) Polyethylene, PVP/Eicosene and mixtures thereof.

4. The method according to one of claims 1 to 3, wherein as fibers polyester fibres, rayon fibres, nylon fibres, cotton fibres, Teflon fibres and preferably Lycra^{®} fibres are used.

5. The method according to one of claims 1 to 4, wherein the used fibers have an average length of between 4 and 4,5 mm.

6. The method according to one of claims 1 to 5, wherein the waxes used in the oil phase are natural or synthetic waxes which are solid at 25°C and below, preferably beeswax, ozokerite, carnauba wax, candelilla wax, wool wax, hard paraffin, ceresin, silicone wax, polyethylene glycol waxes or polyethylene glycol glycol ester waxes.

## Revendications

1. Procédé de fabrication d'un mascara à constituants à base de fibres, **caractérisé en ce qu'**une phase huileuse qui contient des cires, des huiles, des pigments et au moins un polymère ou copolymère soluble dans l'huile ou dispersible dans l'huile, est mélangée à une phase aqueuse de 65 à 78° C jusqu'à émulsification complète et un mélange sans alcool, homogène produit en agitant à 12 à 20 U/min à une température de 18 à 25° C constitué de fibres et de cire synthétique liquide, de polyéthylène liquide ou de mélanges de ceux-ci est mêlé à l'émulsion en agitant de 50 à 70° C.

2. Procédé selon la revendication 1, **caractérisé en ce que** au moine le polymère ou copolymère soluble dans l'huile ou dispersible dans l'huile est choisi dans le groupe comprenant les copolymères d'anhydride d'acide maléique, d'isopropylmaléate et de monomère d'oléfine comportant 30 à 45 atomes de carbone ; les copolymères de vinylpyrrolidone et d'alpha-oléfines à chaîne longue ; les copolymères d'acide adipique avec de l'acide fumarique, acide phtalique et tricyclodécane diméthiconol ; les copolymères d'acide adipique, de cyclohexane-diméthanol, d'anhydride d'acide maléique, de néopentylglycol et de monomères d'anhydride d'acide trimellitique ; les copolymères d'acide adipique et monomères PPG-10 ; le polyéthylène ; les copolymères de butadiène/isoprène ; les copolymères d'éthyl- ou butyl-esters de copolymères de PVM/MA ; le tricontanyl PVP ; l'acide C20-40 (et) le polyéthylène ; le PVP/eicosène ; le bis-diglycéryl-polyacyladipate-1 ; le polyvinyl-octadécyléther ; et leurs mélanges.

3. Procédé selon la revendication 2, **caractérisé en ce qu'**au moins le polymère ou le copolymère soluble dans l'huile ou dispersible dans l'huile est choisi dans le groupe constitué de PVP tricontanyl, d'acide C20-40 (et) de polyéthylène, de PVP/eicosène et leurs mélanges.

4. Procédé selon l'une quelconque des revendications 1 à 3, **caractérisé en ce que** l'on utilise comme fibres, des fibres de polyester, des fibres de rayonne, des fibres de nylon, des fibres de coton, des fibres de téflon et de préférence des fibres de Lycra®.

5. Procédé selon l'une quelconque des revendications 1 à 4, **caractérisé en ce que** l'on utilise comme fibres, celles présentant une longueur moyenne de 4 à 4,5 mm.

6. Procédé selon l'une quelconque des revendications 1 à 5, **caractérisé en ce que** les cires utilisées dans la phase huileuse sont des cires naturelles ou synthétiques qui sont solides à 25° C et à des températures inférieures, de préférence la cire d'abeille, l'ozocérite, la cire de carnauba, la cire de candellila, la cire de laine, la paraffine dure, la cérésine, la cire de silicone, les cires de polyéthylène-glycol et les cires d'esters de polyglycol.
